(19) 

**Europäisches Patentamt**
**European Patent Office**
**Office européen des brevets**

(11) **EP 4 751 750 A1**

(12) **EUROPEAN PATENT APPLICATION**
published in accordance with Art. 153(4) EPC

(43) Date of publication:
**03.06.2026 Bulletin 2026/23**

(21) Application number: **24845406.8**

(22) Date of filing: **10.07.2024**

(51) International Patent Classification (IPC):
**A61M 5/303** (2006.01)     **A61K 35/12** (2015.01)
**A61K 45/00** (2006.01)     **A61P 43/00** (2006.01)

(52) Cooperative Patent Classification (CPC):
**A61K 35/12; A61K 45/00; A61M 5/30; A61P 43/00**

(86) International application number:
**PCT/JP2024/024991**

(87) International publication number:
**WO 2025/023025 (30.01.2025 Gazette 2025/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.07.2023   JP 2023120704**

(71) Applicant: **Daicel Corporation**
**Osaka-shi, Osaka 530-0011 (JP)**

(72) Inventors:
• **SAKAGUCHI, Yuko**
  **Tokyo 108-8230 (JP)**
• **AOKI, Shigehisa**
  **Saga-shi, Saga 840-8502 (JP)**

(74) Representative: **Grünecker Patent- und
Rechtsanwälte
PartG mbB
Leopoldstraße 4
80802 München (DE)**

(54) **INJECTOR, METHOD FOR FORMING VOID USING SAID INJECTOR, AND METHOD FOR REDUCING INTERSTITIAL LIQUID ACCUMULATED IN OBJECT**

(57)     At a minimum, the following is provided. An injector capable of forming a space in a target is provided. The problem is solved by an injector for injecting a liquid and a gas into a target from an injector body without performing injection through a predetermined structure in a state in which the predetermined structure is inserted into the target, the injector including a storage to store the liquid and the gas, a nozzle in communication with the storage and including an ejection port for ejecting the liquid and gas toward the target, and a pressurizer to pressurize the liquid and gas stored in the storage to eject the liquid and gas from the ejection port toward the target when activated. The ratio of the volume of the gas to the total volume of the liquid and gas is more than 60 % and 80 % or less.

*FIG. 1*

EP 4 751 750 A1

**Description**

TECHNICAL FIELD

The present disclosure relates to an injector, a method for forming a space using the injector, and a method for reducing interstitial fluid accumulation in a target.

BACKGROUND

Injectors for injecting a drug solution into an injection target include needle-equipped syringes that inject via a syringe needle and needleless syringes that inject without a syringe needle. In addition, multi-hole syringe needles, catheters equipped with a syringe needle and a drive source, and the like are also used to deliver drug solutions to injection targets.

Among these, needleless syringes may be configured to inject the injection components by applying pressure to a storage chamber containing the injection liquid using pressurized gas, a spring, or electromagnetic force. For example, a configuration has been adopted in which a plurality of nozzle holes is formed inside the syringe body, and pistons that are driven during injection are arranged in correspondence with the nozzle holes (Patent Literature (PTL) 1). This configuration aims to achieve uniform injection into the target by simultaneously spraying the injection liquid from the plurality of nozzle holes. A plasmid containing the luciferase gene has been injected into rats, and highly efficient cell transfer has been achieved.

Furthermore, there is a type of needleless syringe that uses pressurized gas as a power source for ejecting the injection liquid. For example, a pressurizing mode in which a large amount of pressure is applied instantaneously at the beginning of injection, with the pressure then being gradually reduced over 40 ms to 50 ms, has been disclosed (PTL 2).

On the other hand, a method is known in which air is mixed into the drug solution when it is injected. For example, when azacitidine, a drug used to treat myelodysplastic syndrome, is administered subcutaneously using a needle syringe in the usual way without mixing in air, patients may suffer unpleasant side effects, such as redness, pain, or bruising at the injection site. In contrast, it has been reported that administering the drug in the presence of air prevents contact between the epithelium and the drug solution, thereby reducing side effects (Non-patent Literature (NPL) 1).

Also known is a method of injecting a predetermined gas and a solution containing biomolecules into an injection target. This method has been disclosed as increasing the proportion of biomolecules that function in the target of injection (PTL 3).

Lymphedema is a type of interstitial edema. Lymphedema occurs mainly due to the obstruction of lymphatic flow caused by cancer treatment and typically occurs in the hands and feet.

Currently, known treatments for lymphedema include complex treatments that combine compression therapy, exercise therapy, lifestyle guidance, skin care, and lymphatic drainage, as well as surgical treatments that eliminate lymphatic congestion through lymphaticovenous anastomosis.

CITATION LIST

Patent Literature

> PTL 1: JP 2004-358234 A
> PTL 2: US 2005/0010168 A1
> PTL 3: WO 2022/149550 A1

Non-patent Literature

NPL 1: Can. Oncol. Nurs. J., 22(4): 222-34, 2012

SUMMARY

(Technical Problem)

The aims of the present disclosure are at least as follows. An injector capable of forming a space in a target is provided. Also provided is a novel method for forming a space. Also provided is a novel method for reducing interstitial fluid.

(Solution to Problem)

We conducted extensive research to solve the above problems and have discovered that the above problems can be solved by using a predetermined injector.

The primary features of the present disclosure are as follows.

[1] An injector for injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
wherein a ratio of a volume of the gas to a total volume of the liquid and the gas is more than 60 % and 80 % or less.

[2] The injector according to [1], wherein the gas is air.
[3] The injector according to [1] or [2], wherein injection into the target is injection into the target by jet injection.
[4] The injector according to any one of [1] to [3], wherein an ejection speed of the liquid and the gas exceeds 83.3 μL/s.
[5] The injector according to any one of [1] to [4], wherein the liquid contains a biofunctional substance.
[6] The injector according to [5], wherein the biofunctional substance is at least one selected from the group consisting of nucleic acids, peptides, proteins, and low molecular weight compounds.
[7] An injector for reducing interstitial fluid accumulation in a target,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated.

[8] The injector according to [7], wherein a ratio of a volume of the gas to a total volume of the liquid and the gas is 30 % or more and 80 % or less.
[9] The injector according to [7] or [8], wherein the gas is air.
[10] The injector according to any one of [7] to [9], wherein injection into the target is injection into the target by jet injection.
[11] The injector according to any one of [7] to [10], wherein an ejection speed of the liquid and the gas exceeds 83.3 μL/s.
[12] The injector according to any one of [7] to [11], wherein the liquid contains a biofunctional substance.
[13] The injector according to [12], wherein the biofunctional substance is at least one selected from the group consisting of nucleic acids, peptides, proteins, and low molecular weight compounds.
[14] A method for forming a space using an injector,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
the method comprising:

storing the liquid and the gas in the storage;
pressurizing the liquid and the gas stored in the storage; and

injecting the liquid and the gas into the target,
wherein an injection site of the target has a viscoelastic modulus of 47 % or more and 82 % or less at 25 °C.

[15] The method for forming a space according to [14], wherein the injecting is injecting into the target by jet injection.
[16] The method for forming a space according to [14] or [15], wherein a ratio of a volume of the gas to a total volume of the liquid and the gas is 30 % or more and 80 % or less.
[17] The method for forming a space according to any one of [14] to [16], wherein the gas is air.
[18] The method for forming a space according to any one of [14] to [17], wherein an ejection speed of the liquid and the gas exceeds 83.3 μL/s.
[19] The method for forming a space according to any one of [14] to [18], wherein a relaxation time when viscoelasticity is measured at 25 °C at an injection site of the target is 0.89 ms or more and 4.39 ms or less.
[20] A method for reducing interstitial fluid accumulation in a target using an injector,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
the method comprising:

storing the liquid and the gas in the storage;
pressurizing the liquid and the gas stored in the storage; and
injecting the liquid and the gas into the target.

[21] The method according to [20], wherein the target is a mammal other than a human.
[22] The method according to [20] or [21], wherein the injecting is injecting into the target by jet injection.
[23] The method according to any one of [20] to [22], wherein a ratio of a volume of the gas to a total volume of the liquid and the gas is 30 % or more and 80 % or less.
[24] The method according to any one of [20] to [23], wherein the gas is air.
[25] The method according to any one of [20] to [24], wherein an ejection speed of the liquid and the gas exceeds 83.3 μL/s.
[26] The method according to any one of [20] to [25], wherein the liquid contains a biofunctional substance.
[27] The method according to [26], wherein the biofunctional substance is at least one selected from the group consisting of nucleic acids, peptides, proteins, and low molecular weight compounds.
[28] The method according to any one of [20] to [27], further comprising injecting cells or a solution that contains a biofunctional substance into a space formed by the injecting of the liquid and the gas.

(Advantageous Effect)

The present disclosure can at least achieve the effect of enabling formation of a space in a predetermined target. It can also provide a novel method for reducing interstitial fluid.

BRIEF DESCRIPTION OF THE DRAWINGS

In the accompanying drawings:

FIG. 1 is a diagram illustrating a schematic configuration of an injector according to an embodiment of the present disclosure;
FIG. 2 is an observation image, under a stereomicroscope, of a tissue section of Test Example 1 (photograph as a substitute for a drawing);
FIG. 3 is an observation image, under a stereomicroscope, of a tissue section of Test Example 2 (photograph as a substitute for a drawing);
FIG. 4 is an observation image, under a stereomicroscope, of a tissue section of Test Example 3 (photograph as a substitute for a drawing);
FIG. 5 is an observation image, under a stereomicroscope, of a tissue section of Test Example 4 (photograph as a substitute for a drawing);

FIG. 6 is a graph illustrating the relative tail diameters 21 and 28 days after skin peeling;

FIG. 7 is an observation image, under a stereomicroscope, of konjac jelly after injecting liquid and gas at a gas ratio of 50 % (photograph as a substitute for a drawing);

FIG. 8 is an observation image, under a stereomicroscope, of konjac jelly after injecting liquid and gas at a gas ratio of 60 % (photograph as a substitute for a drawing);

FIG. 9 is an observation image, under a stereomicroscope, of konjac jelly after injecting liquid and gas at a gas ratio of 80 % (photograph as a substitute for a drawing);

FIG. 10 is an observation image after injecting liquid and gas into 0.7 % agarose gel at a gas ratio of 80 % (photograph as a substitute for a drawing);

FIG. 11 is an observation image after injecting liquid and gas into 0.5 % agarose gel at a gas ratio of 80 % (photograph as a substitute for a drawing);

FIG. 12 is an observation image after injecting liquid and gas into a marshmallow at a gas ratio of 50 % (photograph as a substitute for a drawing);

FIG. 13 is an observation image after injecting liquid and gas into a marshmallow at a gas ratio of 60 % (photograph as a substitute for a drawing);

FIG. 14 is an observation image after injecting liquid and gas into a marshmallow at a gas ratio of 80 % (photograph as a substitute for a drawing); and

FIG. 15 is an observation image, under a stereomicroscope, of a section after injecting liquid and gas into a pig abdomen at a gas ratio of 50 % (photograph as a substitute for a drawing).

DETAILED DESCRIPTION

The configurations and combinations thereof in each embodiment are merely examples, and additions, omissions, substitutions, and other modifications of the configurations are possible as appropriate within a scope that does not deviate from the spirit of the present disclosure. The present disclosure is not limited by the embodiments, but only by the scope of the claims. Additionally, each feature disclosed herein can be combined with any other feature disclosed herein.

Furthermore, a numerical range expressed using "to" indicates a range that includes the numerical values written before and after "to" as the lower and upper limits, and "A to B" means that the range is A or greater and B or less.

The present disclosure includes an aspect of an injector (first embodiment), an aspect of an injector for reducing interstitial fluid accumulation in a target (second embodiment), an aspect of a method for forming a space using an injector (third embodiment), an aspect of a method for reducing interstitial fluid accumulation in a target using an injector (fourth embodiment), and a method for reducing interstitial fluid accumulation in a target (fifth embodiment).

First Embodiment

A first embodiment of the present disclosure is

an injector for injecting a liquid and a gas into a target from an injector body without performing injection through a predetermined structure in a state in which the predetermined structure is inserted into the target, the injector including:

a storage configured to store the liquid and the gas;

a nozzle in communication with the storage and comprising an ejection port for ejecting the liquid and the gas toward the target; and

a pressurizer configured to pressurize the liquid and the gas stored in the storage to eject the liquid and the gas from the ejection port toward the target when the pressurizer is activated.

In the present embodiment, since the liquid and the gas are present in the storage, the liquid is injected into the target while the gas dissolves in the liquid as pressure is applied. After injection into the target, as the pressure is reduced, some of the gas dissolved in the liquid returns to gas form, generating countless microscopic bubbles, and it is thought that the shearing action of these bubbles can form a plurality of spaces in the target. The spaces usually have a maximum diameter of about 10 $\mu$m to about 1000 $\mu$m, but a larger space may be formed by interconnection among a plurality of spaces. The formation of a space can be confirmed using a stereomicroscope or the like.

The space can be used as a flow path or reservoir for any liquid or gas, for example.

[Liquid]

In the present embodiment, the liquid contained in the storage may be water, for example, and is preferably a liquid that is isotonic with an animal's body fluids; specifically, saline solution, Ringer's solution, lactated Ringer's solution, acetate Ringer's solution, bicarbonate Ringer's solution, and the like can be suitably used. These can be prepared by known methods, and commercially available products can also be used.

The liquid that can be injected by the injector of the present embodiment may as needed contain conventional additives, such as biofunctional substances, buffers, tonicity agents, pH adjusters, antioxidants, thickeners, stabilizers, humectants, emulsifiers, and binders, or contain oils and fats. A space can still be formed in a specified target and interstitial fluid reduced, however, even if none of these is contained.

The biofunctional substance is not particularly limited as long as it exhibits physiological activity in the target. The biofunctional substance may be one type or a plurality of types. The biofunctional substance may be a natural product or an artificially synthesized product.

The biofunctional substance is preferably at least one selected from the group consisting of nucleic acids, peptides, proteins, and low molecular weight compounds.

Nucleic acids include, for example, DNA, RNA and PNA. Furthermore, the nucleic acid may be a nucleic acid containing a portion that encodes a protein or may be a nucleic acid that does not contain a portion that encodes a protein (a non-coding nucleic acid).

Examples of peptides and proteins include antigens (i.e., substances that induce the production of antibodies against the peptide or protein), antibodies, peptide vaccines, protein vaccines, peptide hormones, protein hormones, growth factors, cytokines, blood coagulation factors, serum albumin, digestive enzymes, anti-inflammatory peptides, and anti-inflammatory proteins.

The protein may also be an extracellular matrix. The extracellular matrix is a non-cellular component that constitutes the human body and is expected to contribute to maintaining spaces as a scaffold for cells. Examples of the extracellular matrix include collagen, fibronectin, and laminin.

Low molecular weight compounds generally refers to compounds having a molecular weight of 2000 or less, but low molecular weight compounds are not limited to this range and include compounds industrially considered as low molecular weight compounds. The molecular weight of the low molecular weight compound is preferably in the range of 50 or more, or 100 or more, for example. Other example ranges include 2000 or less and 1000 or less. That is, example ranges include 50 to 2000, 50 to 1000, and 100 to 2000.

In the present disclosure, physiological activity refers to an effect on a specific physiological regulatory function of a living organism. The evaluation index for physiological activity can be appropriately set depending on the purpose and may be either a qualitative or quantitative index, with a quantitative index being preferred. For example, the amount of a particular mRNA, protein, cytokine, antibody titer, or number of cells of a particular cell type can be used as an index. These quantitative indicators can be quantified by methods known in the art.

In particular, when the biofunctional substance is a nucleic acid containing a portion encoding a protein, the amount of the protein encoded by the nucleic acid can be quantified and used as an evaluation index for physiological activity. The activity of the protein may also be quantitatively evaluated. For example, when the protein is luciferase, the physiological activity can be evaluated by measuring the intensity of bioluminescence.

When the biofunctional substance is a nucleic acid, the nucleic acid may be incorporated into a viral vector or carried on lipid nanoparticles and contained in the liquid, but viral vectors or lipid nanoparticles do not have to be used. When viral vectors or lipid nanoparticles are not used, the possibility of adverse reactions such as anaphylaxis being induced in the target can be reduced.

The content of the biofunctional substance relative to the total volume of the liquid can be appropriately set based on the type of the biofunctional substance, the target, the physiological activity exhibited by the biofunctional substance in the target into which the biofunctional substance is injected, and the like.

Examples of buffers that can be used include buffers using phosphate (e.g., phosphate buffer, phosphate buffered saline (PBS) (which may be PBS(+) or PBS(-)), Dulbecco's phosphate buffered saline (D-PBS), citrate-phosphate buffer, citrate-phosphate buffered saline, and the like), citrate buffer, tris(hydroxymethyl)aminomethane-HCl buffer (tris-hydrochloric acid buffer), acetate buffer, GOOD buffer (e.g., HEPES-NaOH buffer and the like), amino acid buffer (e.g., glycine-hydrochloric acid buffer, glycine-NaOH buffer, glycylglycine-KOH buffer, and the like), imidazole buffer, and the like.

Among these, a buffer solution using phosphate is preferred from the perspective of versatility.

Examples of the tonicity adjusting agent include ionic tonicity adjusting agents and non-ionic tonicity adjusting agents.

Examples of ionic tonicity agents include salts such as sodium chloride, potassium chloride, calcium chloride, and magnesium chloride.

Examples of non-ionic tonicity agents include glycerin, propylene glycol, polyethylene glycol, glucose, sorbitol, mannitol, trehalose, maltose, and sucrose.

Among these, sodium chloride is preferred from the perspective of versatility.

Examples of pH adjusters include hydrochloric acid, phosphoric acid, citric acid, acetic acid, sodium hydroxide, potassium hydroxide, sodium carbonate, and sodium hydrogen carbonate.

Examples of antioxidants include ascorbic acid, sodium sulfite, butylhydroxyanisole, butylhydroxytoluene, propyl gallate, and tocopherol.

Thickeners include alginic acid, polyethylene glycol, hydroxypropyl methylcellulose, sodium carboxymethylcellulose, and the like.

Examples of fats and oils include sesame oil, soybean oil, rapeseed oil, and olive oil. The glycerin constituting fats and oils can also be used. These oils and fats are expected to remain in the formed spaces and contribute to maintaining the spaces.

[Gas]

In the present embodiment, the gas stored in the storage is not particularly limited but may be air. The air may be any commonly used air, and its composition is not particularly limited. For example, a gas mixture of approximately 80 % nitrogen and approximately 20 % oxygen can be used. Examples of the gas include nitrogen, oxygen, ozone, carbon dioxide, hydrogen, and carbon monoxide, along with a mixture of two or more of these. In a preferred embodiment, the gas does not contain microorganisms or the like, or even if the gas does contain microorganisms or the like, the microorganisms or the like are dead.

The ratio of the volume of the gas to the total volume of the liquid and the gas is not particularly limited but is preferably 30 % or more, and more preferably more than 60 %. This ratio is preferably less than 100 % and more preferably 80 % or less. That is, preferred ranges for the ratio of the volume of the gas include 30 % or more and less than 100 %, 30 % or more and 80 % or less, and more than 60 % and 80 % or less. If the volume of the gas is in this range, a plurality of spaces is likely to form in the target.

[Target]

The target that can be injected by the injector according to the present embodiment may be either a living organism or a non-living material. The target in the present embodiment may, for example, be one or more selected from the group consisting of cells, cell sheets, cell masses, tissues, organs (skin, internal organs, and the like), organ systems, individuals (living organisms), and the like. Furthermore, the target may be one or more selected from the group consisting of tissue, organs (skin, internal organs, and the like), organ systems, individuals (living organisms), and the like. The system may be any of an in vitro system, an in vivo system, an ex vivo system, and the like. The cell mass may be a cell mass obtained by three-dimensional culture, and the organ (skin, internal organs, and the like) may be an organoid.

Furthermore, when the target is injected, injection may be performed into a lower level contained therein. That is, for example, when the target is an individual (living organism), injection may be performed into a tissue contained in the individual (living organism), into a cell contained in the individual (living organism), or into both. Furthermore, when the target is a tissue, injection may be performed into cells contained in the tissue, into the extracellular matrix contained in the tissue, or into both.

Furthermore, when the target in the present embodiment is one or more selected from the group consisting of cells, cell sheets, cell masses, tissues, organs (skin, internal organs, and the like), and organ systems, the target may be one or more selected from the group consisting of cells, cell sheets, cell masses, tissues, organs (skin, internal organs, and the like), and organ systems present in an individual (living organism), or the target may be one or more selected from the group consisting of cells, cell sheets, cell masses, tissues, organs (skin, internal organs, and the like), and organ systems not present in an individual (living organism) (for example, having been extracted or separated from an individual (living organism) or having been produced outside an individual (living organism)).

Furthermore, the target in the present embodiment may be one or more selected from the group consisting of cells, cell sheets, cell masses, tissues, organs (skin, internal organs, and the like), and organ systems derived from stem cells such as iPS cells (induced pluripotent stem cells), and these may be present in an individual (living organism) or not present in an individual (living organism) (for example, having been extracted or separated from an individual (living organism), or having been produced outside an individual (living organism)).

The individual (living body) is preferably an individual mammal (living body). The mammal is not particularly limited, and examples thereof include humans and mammals other than humans. Examples of mammals other than humans include mice, rats, guinea pigs, hamsters, cows, goats, sheep, pigs, monkeys, dogs, and cats.

The organ may be one or more selected from the group consisting of joint cavities, eyeballs, skin, muscles, bones, cartilage, bone marrow, ligaments, brain, spinal cord, lungs, liver, heart, kidneys, pancreas, gallbladder, digestive tract, urinary bladder, reproductive organs, lymph nodes, lymphatic network, and blood vessels, and a tumor occurring in any one of these. Reproductive organs include the testes and ovaries.

Furthermore, regardless of whether the target of the present embodiment is any of the above, when injection is performed into a cell, injection may be performed into the cytoplasm of the cell, injection may be performed into the cell nucleus of the cell, or injection may be performed into both the cytoplasm and the cell nucleus of the cell.

Furthermore, the target in the present embodiment is not particularly limited but is preferably one or more selected from the group consisting of the intradermal space, the subcutaneous space, and the underlying muscle layer inside the skin of an individual mammal (living organism). In this case, a method can be employed in which the liquid and the gas are injected into the skin by ejecting them from an injector toward the surface of the skin, and then into one or more selected from the group consisting of the intradermal space, the subcutaneous space, and the underlying muscle layer inside the skin.

In particular, the target in the present embodiment is preferably a site in the skin of an individual mammal (living organism) where interstitial edema occurs.

When the injector of the present embodiment is used to inject a liquid and a gas into a site where interstitial edema has occurred, a plurality of spaces is created. It is thought that these spaces act as channels for interstitial fluid, reducing the amount of interstitial fluid that has accumulated locally.

The injection site of the target preferably has a viscoelastic modulus at 25 °C of 47 % or more and 82 % or less. The viscoelastic modulus is a value obtained by the following equation and indicates the ratio between the viscous element and the elastic element of the target.

Viscoelastic modulus = (viscous strain amount)/(viscous strain amount + elastic strain amount)

An ideal elastic body has a value of 0 %, and an ideal viscous body has a value of 100 %.

The viscoelastic modulus of the target at 25 °C is more preferably 47 % or more and 71 % or less.

The relaxation time when viscoelasticity is measured at 25 °C at the injection site of the target is 0.89 ms or more and 4.39 ms or less, more preferably 0.89 ms or more and 3.11 ms or less.

The relaxation time is the time constant of the stress relaxation behavior of a viscoelastic body and is an index that represents the vibration frequency characteristics of the viscoelastic material. This value can be calculated using the following equation.

$$\text{Relaxation time} = (\text{viscosity data})/(\text{elasticity data})$$

The viscoelastic modulus and relaxation time can be measured according to the instruction manual for a Vesmeter (for biometric measurements, E-100HS, WAVECYBER CORP.). Specifically, at room temperature (25 °C), the probe of the Vesmeter is placed vertically on the measurement target from above, the same location is measured 4 to 35 times, and the average value can be used as the measured value.

[Injector]

As described above, the injector of the present embodiment includes a storage configured to store a liquid and a gas, a nozzle in communication with the storage and including an ejection port for ejecting the liquid and the gas toward the target, and a pressurizer configured to pressurize the liquid and the gas stored in the storage to eject the liquid and the gas from the ejection port toward the target when the pressurizer is activated. By ejecting the liquid and the gas from the ejection port of the nozzle toward the target, the injector can inject the liquid and the gas into the target.

The injector of the present embodiment injects the liquid and the gas into the target from the injector body without performing injection through a predetermined structure in a state in which the predetermined structure is inserted into the target. The injector according to the present embodiment may include a predetermined structure, such as a catheter, to guide the liquid and the gas from the injector body to the target when, for example, the injector body and the target are separated by a large distance. Therefore, the injector according to the present embodiment may or may not include such a predetermined structure.

In the injector according to the present embodiment, the energy imparted by the pressurizer to pressurize the liquid and gas can be imparted in the form of energy imparted by a known pressurizing technique. An example of the energy to be applied may be chemically generated energy, such as combustion energy generated by an oxidation reaction of explosives, gunpowder, or the like. Alternatively, the energy for applying pressure may be generated electrically, for example, by a piezoelectric element or an electromagnetic actuator driven by input power. As another alternative, the energy for pressurization may be generated physically, examples of which include elastic energy of an elastic body or internal energy of a compressible body such as compressed gas. That is, the energy for pressurization may be any energy that enables the ejection of the liquid from the injector. The energy for pressurization may be a composite energy that is an appropriate combination of forms of internal energy such as combustion energy, electrical energy, and elastic energy.

In view of the above, the pressurizer may, for example, apply pressure by utilizing the pressure generated by the combustion of an explosive ignited by an ignition device, or by utilizing the pressure generated when compressed gas is released. The pressurizer may apply pressure using the biasing force of a compression spring or may apply pressure using electromagnetic force, such as by using a linear electromagnetic actuator. The pressurizer preferably uses at least the pressure generated by the combustion of an explosive ignited by the ignition device and may also use any of the other pressurizing modes described above in combination.

In a case in which the pressurizer uses the pressure generated by the combustion of an explosive, the explosive may be, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of a combination of two or more of these. A characteristic of these types of explosive is that although their combustion products are gaseous at high temperatures, they do not contain gaseous components at room temperature, so that the combustion products condense immediately after ignition.

Furthermore, when the pressurizer uses the energy generated by the combustion of the gas generating agent as the ejection energy, it is also possible to use, as the gas generating agent, single-base smokeless powder (GG) or various gas generating agents used in gas generators for airbags and gas generators for seatbelt pretensioners. An example of a single-base smokeless powder is a single-base smokeless powder containing 98 mass% of nitrocellulose, 0.8 mass% of diphenylamine, and 1.2 mass% of potassium sulfate.

Hereinafter, a syringe 1 (needleless syringe) will be described as an example of the injector of the present embodiment with reference to the drawings. The following configuration is an example and is not limiting. The terms "tip side" and "base side" are used to indicate the relative positional relationship in the longitudinal direction of the syringe 1. The "tip side" refers to a position closer to the tip of the syringe 1 described below, i.e., closer to an ejection port 31a, and the "base side" refers to the direction opposite to the "tip side" in the longitudinal direction of the syringe 1, i.e., the direction toward a drive unit 7. Furthermore, although combustion energy of an explosive ignited by an ignition device is used as ejection energy for pressurization in the illustrated example, the present disclosure is not limited to this configuration.

(Configuration of syringe 1)

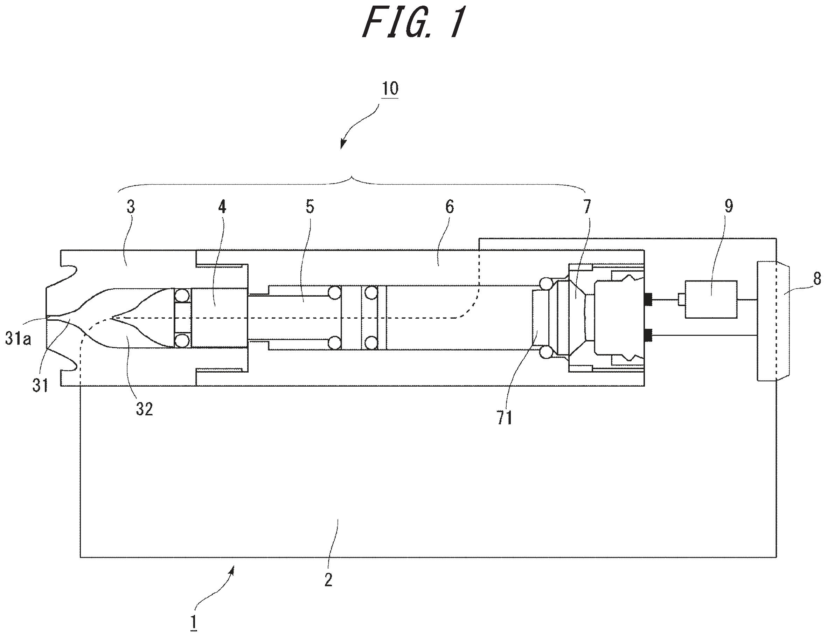

FIG. 1 is a diagram illustrating the schematic configuration of the syringe 1 and is also a cross-sectional view of the syringe 1 taken along its longitudinal direction. The syringe 1 is obtained by integrating a subassembly, formed by a syringe portion 3 and a plunger 4, and a subassembly, formed by a syringe body 6, a piston 5, and the drive unit 7, into a syringe assembly 10, and then attaching the syringe assembly 10 to a housing (syringe housing) 2.

As described above, the syringe assembly 10 is configured to be detachable from the housing 2. A storage 32, formed between the syringe portion 3 and the plunger 4 included in the syringe assembly 10, is filled with a liquid and a gas. The syringe assembly 10 is a disposable unit that is disposed of each time the liquid and the gas are ejected. The storage 32 may be composed of a first storage for storing the gas and a second storage for storing the liquid. On the other hand, the housing 2 includes a battery 9 that supplies power to an igniter 71 included in the drive unit 7 of the syringe assembly 10. Power is supplied from the battery 9 via wiring between an electrode on the housing 2 side and an electrode on the drive unit 7 side of the syringe assembly 10 when the user presses a button 8 provided on the housing 2. The shape and position of the electrode on the housing 2 side and the electrode on the drive unit 7 side of the syringe assembly 10 are designed so that the electrodes come into contact automatically when the syringe assembly 10 is attached to the housing 2. The housing 2 is a unit that can be reused repeatedly as long as the battery 9 has enough power to supply to the drive unit 7. In the case of the housing 2, when the battery 9 runs out of power, it is possible to replace only the battery 9 and continue to use the housing 2.

Next, the syringe assembly 10 will be described in detail. First, the subassembly including the syringe portion 3 and the plunger 4 will be described. The syringe portion 3 has the storage 32 formed therein, which is a space capable of storing the liquid and the gas. More specifically, as illustrated in FIG. 1, the plunger 4 is slidably disposed along the inner wall surface of the syringe portion 3 extending in the axial direction, and the inner wall surface of the syringe portion 3 and the plunger 4 define the storage 32. The syringe portion 3 also has a nozzle 31 that communicates with the storage 32, and the ejection port 31a is formed at the tip side of the nozzle 31. The nozzle 31 has a flow path cross-sectional area that gradually decreases from the storage 32 side toward the ejection port 31a side. The nozzle 31 is a flow path for guiding the liquid and gas filled in the storage 32 to the ejection port 31a. In the example illustrated in FIG. 1, the shape of the tip side of the plunger 4 roughly matches the shape of the nozzle 31.

Next, the subassembly including the syringe body 6, the piston 5, and the drive unit 7 will be described. The piston 5 is made of metal, for example, and by being pressurized by a combustion product (combustion gas) generated by the igniter 71 of the drive unit 7, the piston 5 is configured to slide through a through-hole formed inside the syringe body 6. The syringe body 6 is a generally cylindrical member, and the piston 5 is housed in the syringe body 6 in a manner allowing the piston 5 to slide freely along the inner wall surface extending in the axial direction of the syringe body 6. The piston 5 may be made of resin, and in this case, metal may be used in combination in the portions that require heat resistance and pressure resistance. As illustrated in FIG. 1, the piston 5 is integrally connected to the plunger 4.

Next, the drive unit 7 will be described. As illustrated in FIG. 1, the drive unit 7 is fixed to the base side of the syringe body 6, with the through-hole in the syringe body 6 as a reference. The drive unit 7 has an igniter 71 that is an electric igniter. The igniter 71 is disposed so as to face the inside of the through-hole in the syringe body 6 and contains an ignition charge therein. As the ignition charge, various types of explosives can be used as described above. The ignition charge may be contained in a powder cup made of suitable thin metal, for example.

In the syringe 1 configured as described above, the volume of the gas stored in the storage 32 is adjusted to be, for example, 30 % or more and 80 % or less of the volume of the storage 32.

Next, the operation of the syringe 1 having the above configuration will be described. As illustrated in FIG. 1, with the syringe assembly 10 attached to the housing 2, liquid and gas can be drawn through the ejection port 31a of the nozzle 31. This allows the storage 32 to be filled with the liquid and the gas. From this state, for example, when the user presses the button 8 on the housing 2 while the ejection port 31a of syringe 1 is in contact with the target, this triggers the supply of operating power from battery 9 to the igniter 71 of drive unit 7 and activates the igniter 71. When the igniter 71 is activated, the ignition charge is ignited and burns, generating combustion products (such as a flame and combustion gases). As a result, for example, the powder cup of the igniter 71 is split open, and the combustion gas of the ignition charge is released into the through-hole in the syringe body 6. This causes the pressure inside the through-hole of the syringe body 6 to increase suddenly, pressing the piston 5 toward the tip side of the syringe body 6, which causes the piston 5 to slide toward the tip side along the inner wall surface of the through-hole in the syringe body 6. As described above, since the plunger 4 is integrally connected to the piston 5, the plunger 4 also slides along the inner wall surface of the syringe portion 3 in conjunction with the piston 5. That is, when the plunger 4 is pushed toward the nozzle 31 located at the tip side of the syringe portion 3, the volume of the storage 32 containing the liquid and the gas decreases, and the storage 32 is suddenly pressurized. As a result, the liquid and gas filling the storage 32 are forced into the nozzle 31 and ejected at high pressure from the ejection port 31a. The liquid and the gas can thus be injected into the target.

Furthermore, although no additional explosive components are particularly disposed within the syringe body 6 illustrated in FIG. 1, to adjust the transition of pressure applied to the liquid and the gas via the piston 5, a gas generating agent or the like that generates gas upon burning due to the combustion products produced by the combustion of the explosive in the igniter 71 can be disposed within the igniter 71 or the through-hole of the syringe body 6. The configuration in which a gas generating agent is disposed inside the igniter 71 is a known technique as disclosed in WO 2001-031282 A1 and JP 2003-25950 A. An example of the gas generating agent is a single-base smokeless powder containing 98 % by mass of nitrocellulose, 0.8 % by mass of diphenylamine, and 1.2 % by mass of potassium sulfate. Various gas generating agents used in gas generators for airbags and gas generators for seatbelt pretensioners can also be used. By adjusting the dimensions, size, shape, and particularly the surface shape of the gas generating agent when it is placed in the through-hole, the time it takes for the gas generating agent to complete combustion can be changed. This allows the pressure transition applied to the liquid and the gas to be a desired transition, that is, a transition that allows the liquid and the gas to reach the target appropriately. In the present embodiment, the drive unit 7 also includes a gas generating agent and the like that are used as needed. In the present embodiment, the plunger 4 and the piston 5 are included in the "pressurizer".

In the present embodiment, injecting the liquid and the gas into the target using an injector may be injecting the liquid and the gas into the target by jet injection.

Here, in the present disclosure, "jet injection" refers to an injection characterized in that a liquid and a gas are ejected from an ejection port toward a target, thereby forming a through-hole that penetrates the boundary between the inside and outside of the target, and a high-pressure ultra-fine liquid stream that can inject the liquid and the gas into the target through the through-hole is generated. For example, when the target is an individual mammal (living organism), jet injection refers to an injection characterized by producing a high-pressure ultra-fine liquid stream that can, for example, penetrate the skin of the individual mammal (living organism).

The injector according to the present embodiment can eject the liquid and the gas at an ejection speed typically exceeding 83.3 $\mu$L/s. The ejection speed is preferably 200 $\mu$L/s or more, more preferably 250 $\mu$L/s or more, even more preferably 300 $\mu$L/s or more, still more preferably 350 $\mu$L/s or more, yet more preferably 400 $\mu$L/s or more, and most preferably 500 $\mu$L/s or more. No upper limit is established, but an example is 5000 $\mu$L/s or less. That is, examples of preferred ranges of the ejection speed include more than 83.3 $\mu$L/s and 5000 $\mu$L/s or less, 200 $\mu$L/s or more and 5000 $\mu$L/s or less, 250 $\mu$L/s or more and 5000 $\mu$L/s or less, 300 $\mu$L/s or more and 5000 $\mu$L/s or less, 350 $\mu$L/s or more and 5000 $\mu$L/s or less, 400 $\mu$L/s or more and 5000 $\mu$L/s or less, and 500 $\mu$L/s or more and 5000 $\mu$L/s or less.

When the ejection speed is within the above ranges, injection into the target can be easily performed as jet injection.

In the present disclosure, the ejection speed is the speed of the liquid upon ejection from the ejection port and can be calculated by, for example, dividing the ejected volume by the time required from the start to the end of ejection. The time required from the start to the end of ejection can be measured by, for example, capturing an image of the liquid being ejected from the ejection port using an imaging device such as a high-speed camera.

The ejection speed can be set within the above ranges by adjusting the ejection energy according to the shape and material of the nozzle, the volume and viscosity of the liquid, and the like.

The amount of the liquid to be injected into the target is determined according to the volume of the storage and the volume of the gas stored in the storage.

The amount of the liquid injected into the target may, for example, be 10 $\mu$L or more, 50 $\mu$L or more, or 100 $\mu$L or more. Other examples include 50 mL or less, 10 mL or less, and 5 mL or less. That is, examples include 10 $\mu$L to 50 mL, 50 $\mu$L to 10 mL, and 100 $\mu$L to 5 mL.

The time required to inject the liquid into the target is not particularly limited as long as this time suffices for ejection at the ejection speed of the amount of liquid to be injected, but examples include 0.001 seconds or more, 0.005 seconds or more, or 0.01 seconds or more. Other examples include 100 seconds or less, 50 seconds or less, and 10 seconds or less. That is, examples include 0.001 seconds to 100 seconds, 0.005 seconds to 50 seconds, and 0.01 seconds to 10 seconds.

<Second Embodiment>

A second embodiment of the present disclosure is

an injector for reducing interstitial fluid accumulation in a target,
the injector injecting a liquid and a gas into a target from an injector body without performing injection through a predetermined structure in a state in which the predetermined structure is inserted into the target, the injector including:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated.

When lymphatic vessels are partially removed or damaged during surgery for cancer treatment, the flow of lymph (also known as interstitial fluid) is impeded, leading to local accumulation, which can cause interstitial edema. This condition is called lymphedema.

When the injector of the present embodiment is used to inject a liquid and a gas into a site where interstitial edema has occurred, a plurality of spaces is created. It is thought that these spaces act as channels for interstitial fluid, reducing the amount of interstitial fluid that has accumulated locally. In this manner, the injector of the present embodiment can be used as an injector to reduce interstitial fluid accumulation in a target. Furthermore, when the interstitial fluid is derived from lymphatic vessels, the injector can also be used as an injector for treating lymphedema.

The liquid, gas, target, and injector in the present embodiment are the same as those in the first embodiment. When the liquid contains a biofunctional substance, the biofunctional substance is preferably collagen.

<Third Embodiment>

A third embodiment of the present disclosure is

a method for forming a space using an injector,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
the method comprising:

storing the liquid and the gas in the storage;
pressurizing the liquid and the gas stored in the storage; and
injecting the liquid and the gas into the target,

wherein the target has a viscoelastic modulus of 47 % or more and 82 % or less at 25 °C.

The liquid, gas, target, and injector in the present embodiment are the same as those in the first embodiment.

The process of storing the liquid and the gas in the storage is not particularly limited but may be a process of storing the liquid and the gas in the storage by drawing them from outside the injector through the ejection port of the nozzle.

The energy imparted by the pressurizer in the step of pressurizing the liquid and the gas stored in the storage can be in the form of energy imparted by a known pressurizing technique. Specifically, the description of the injector of the first embodiment may be cited.

The process of injecting the liquid and the gas into the target may, for example, be carried out by bringing the ejection port into contact with the target and ejecting the liquid and the gas through the nozzle using the ejection energy generated by the process of pressurizing the liquid and the gas.

The steps of storing the liquid and the gas in the storage, pressurizing the liquid and the gas stored in the storage, and injecting the liquid and the gas into the target may be performed once or multiple times. Furthermore, when the process of injection is performed multiple times, the target injection sites may be the same or different.

<Fourth embodiment>

A fourth embodiment of the present disclosure is

a method for reducing interstitial fluid accumulation in a target using an injector,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
the method comprising:

storing the liquid and the gas in the storage;
pressurizing the liquid and the gas stored in the storage; and
injecting the liquid and the gas into the target.

When the interstitial fluid is derived from lymphatic vessels, the method according to the present embodiment may be a method for treating lymphedema.

The liquid, gas, target, and injector in the present embodiment are the same as those in the first embodiment.

The explanation of the third embodiment may be cited with reference to the steps of storing the liquid and the gas in the storage, pressurizing the liquid and the gas stored in the storage, and injecting the liquid and the gas into the target.

The reduction method according to the present embodiment may further include injecting cells or a solution that contains a biofunctional substance into a space formed by the injecting of the liquid and the gas.

The spaces formed by the process of injection can be used as reservoirs for cells or a solution that contains a biofunctional substance, allowing these solutions and the like to be distributed and retained locally.

The solution containing the biofunctional substance may be a solution yielded by dissolving (in the present disclosure, this includes suspension and emulsification) the biofunctional substance that can be used in the first embodiment in a liquid that can be used in the first embodiment. When the liquid contains a biofunctional substance, the biofunctional substance contained in the liquid and the biofunctional substance contained in the solution may be the same or different.

The cells used in the process of injecting cells or a solution that contains a biofunctional substance may be cells extracted from a living organism or may be cultured cells derived from stem cells such as iPS cells (induced pluripotent stem cells). The cells may also be in the form of a cell sheet, cell mass, or the like. The cell mass may be a cell mass obtained by a three-dimensional culture.

The process of injecting the cells or a solution that contains a biofunctional substance may be carried out using the above-described injector or another injector. Other injectors include, but are not limited to, an injector consisting of a syringe, a plunger, and a needle.

The process of injecting cells or a solution that contains a biofunctional substance into the space formed by the process of injection may be performed once or multiple times.

<Fifth Embodiment>

A fifth embodiment of the present disclosure is
a method for reducing interstitial fluid accumulation in a target in need of treatment, the method comprising injecting a composition containing a liquid and a gas into an injection site in the target.

In the present embodiment, the injection can be performed using a needleless syringe. The injection can be performed using the injector in the first embodiment.

In the present embodiment, the liquid may be pressurized. The method according to the present embodiment may further include pressurizing the liquid and the gas in an injector.

The liquid, gas, target, and injector in the present embodiment are the same as those in the first embodiment.

EXAMPLES

The present disclosure will be specifically described below with reference to examples. However, the present disclosure is not limited to the forms of the following Examples.

[Experiment 1: Creation of intradermal spaces in a rat or pig]

(Target of administration)

Rat: an SD rat, female, 8 weeks old was used. After the rat was anesthetized and shaved, it was placed with its flank facing upwards. A drug was then administered into the flank while the rat was held in place.

Pig: an SPF domestic pig was used. After the pig was anesthetized and shaved, it was placed with the abdomen facing upwards. A drug was then administered into the abdomen or groin.

(Liquid and gas filling and administration method)

The injector used had the structure illustrated in FIG. 1. A PBS (Nacalai Tesque, 14249-95) solution was drawn up from the nozzle of the injector into the storage of the injector. The plunger was then further pulled up without drawing up any PBS solution, and the injector was filled with normal laboratory air. The volumes of liquid and gas drawn up are listed in Table 1. After confirming that air was present on the plunger side and the PBS solution was present on the tip side of the nozzle, the solution was administered to the target.

[Table 1]

Table 1

|  | Target | Gas | Liquid | Gas ratio |
|---|---|---|---|---|
| Test Example 1 | rat | 9 μL | 21 μL | 30% |
| Test Example 2 | rat | 15 μL | 15 μL | 50% |
| Test Example 3 | pig | 45 μL | 105 μL | 30% |
| Test Example 4 | pig | 75 μL | 75 μL | 50% |

After administration, the animals were euthanized by exsanguination under anesthesia.

The skin was collected centering on the administration site and was fixed in 10 % neutral buffered formaldehyde solution (Nacalai Tesque, 37152-51) for 1 to 3 nights. Thereafter, the skin was washed twice with PBS and immersed in fresh PBS. The skin was removed from the PBS and immersed in ethanol for dehydration and degreasing. Thereafter, substitution with xylene and paraffin was carried out. The tissue was embedded in paraffin and then thinly sectioned. The obtained sections were deparaffinized using xylene and ethanol. The sections were stained with hematoxylin, washed with water, stained with eosin, and decolorized with ethanol. Dehydration was carried out using ethanol. After replacement with xylene, the sections were mounted on slide glasses using a mounting medium. Microscopic observation was then carried out.

Images of the sections were captured using a Keyence all-in-one fluorescence microscope (BZ-X710).

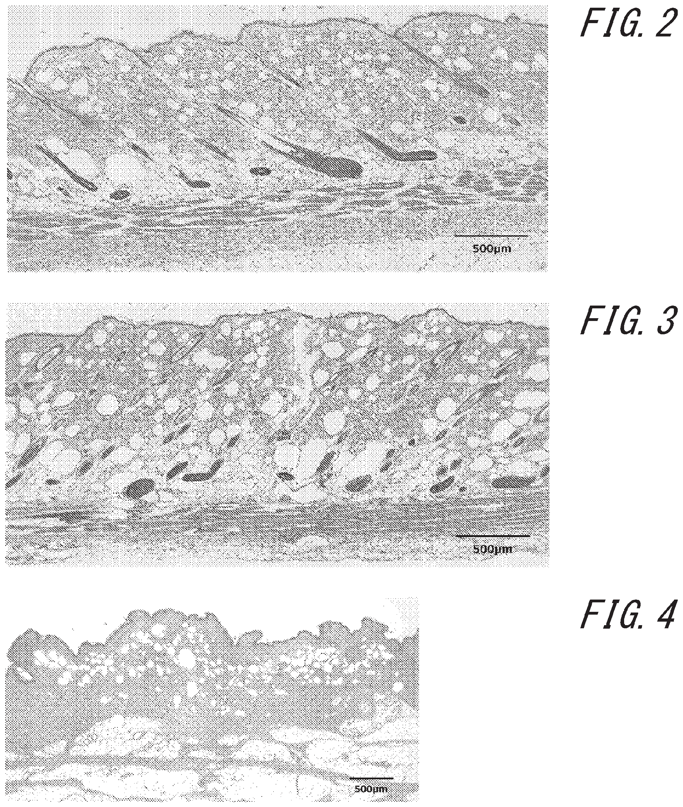

As illustrated in FIGS. 2 to 5, it was confirmed by image observation that spaces were formed throughout the entire dermal layer.

[Experiment 2: Administration to rat tail edema]

Rats were anesthetized, and the skin at a position 10 mm from the base of the tail was peeled in a full-thickness strip in the longitudinal direction over a range of 5 mm and excised. Thereafter, the lymphatic vessel and vein located caudally to the peeling site were cauterized using an electric scalpel. The treated rats were kept until edema formation was confirmed.

Liquid and gas were administered to four locations at the site where the skin had been peeled, shifting by 90 degrees each time in the circumferential direction. The volumes of the liquid and gas were 30 $\mu$L and 20 $\mu$L (gas ratio 40 %), respectively.

The rats were euthanized and sampled 28 days after administration.

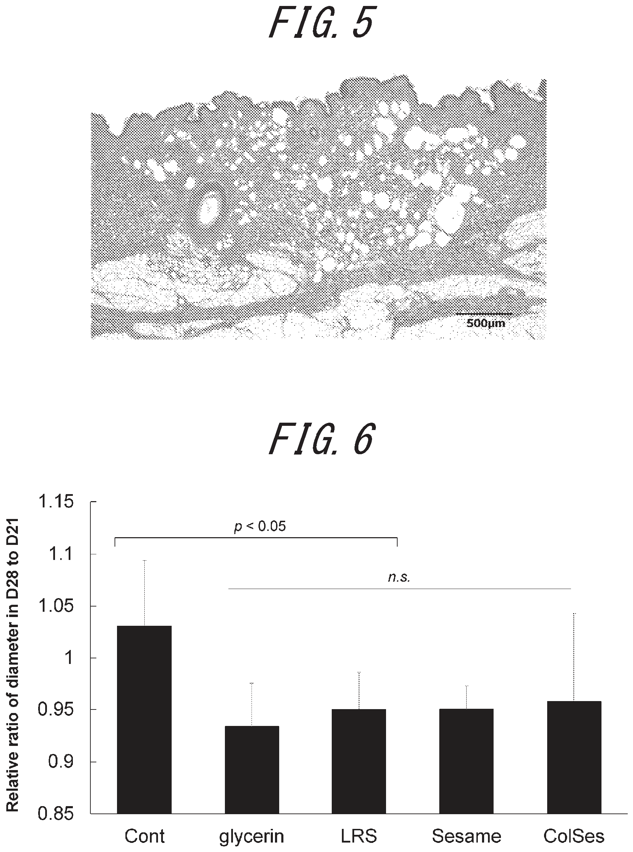

On the 21st, 22nd, and 23rd days after formation of the tail skin peeling site, lactated Ringer's solution (LRS), lactated Ringer's solution containing 5 % glycerin (glycerin), lactated Ringer's solution containing 5 % sesame oil (sesame), or collagen solution containing 5 % sesame oil (ColSes) was injected. Here, the collagen solution was prepared by mixing Nippon Ham pig skin atelocollagen solution (concentration 1 %), 10x Minimum Essential Medium (MEM), and reconstitution buffer solution (2.2 g of sodium bicarbonate and 4.77 g of HEPES dissolved in 100 mL of 0.05 N aqueous NaOH solution) in a ratio of 8:1:1. The value of the diameter at the distal 5 mm position was compared between days 21 and 28 after skin peeling. The results are illustrated in FIG. 6.

The tail diameter was significantly smaller in all injected groups compared with the non-injected group (Cont). Error bars indicate standard deviation. The number of samples was 9 for Cont, 9 for Glycerin, 6 for LRS, 6 for Sesame, and 6 for ColSes.

This suggests that lymphedema improved when 30 $\mu$L each of lactated Ringer's solution, glycerin, sesame oil, and collagen was mixed with 20 $\mu$L of air and injected.

[Experiment 3: Formation of spaces in various administration targets]

(Target of administration)

Mouse: a Bulb/c mouse, female, 8 weeks old was used. After the mouse was anesthetized and shaved, it was placed facing upwards. Drug administration or measurement was then performed on the flank while the mouse was held in place.

Pig: an SPF domestic pig was used. After the pig was anesthetized and shaved, it was placed with the abdomen facing upwards. Drug administration or measurement was then performed on the abdomen or groin. Alternatively, after shaving, the pig was placed face down and the administration or measurement was performed on its back. Abdominal administration or measurement was either performed under normal conditions or with air introduced into the abdominal cavity and the skin stretched. In tables and drawings, the former is indicated as "(without air)" and the latter as "(with air)."

Konjac jelly: Konnyaku Batake® (Konnyaku Batake is a registered trademark in Japan, other countries, or both), Mannan Life. During the physical property measurements, one sheet of PROWIPE (Daio Paper Corporation) was placed on the konjac jelly, and measurements were taken through it.

Agarose gel (0.5 %, 0.7 %, or 1 %): Agarose (Agarose ME (medium electro-osmosis) Classic Type 100G, Nacalai Tesque, 01158-56) was added to PBS to achieve the above concentration, heated in a microwave oven, and then allowed to stand at 4 °C to prepare the gel.

Marshmallow: White marshmallows (Eiwa Corporation) were the target of administration.

(Method of measuring physical properties)

Measurements were carried out according to the instruction manual for a Vesmeter (for biometric measurements, E-100HS, WAVECYBER CORP.). Specifically, at room temperature (25 °C), the probe of the Vesmeter was placed vertically on the measurement target from above, the same location was measured 4 to 35 times, and the average value was used as the measured value.

(Liquid and gas filling and administration method)

The injector used had the structure illustrated in FIG. 1. A predetermined amount of D-PBS(-) (Nacalai Tesque, 14249-24) solution containing 1 % malachite green (Malachite Green Oxalate, manufactured by Nacalai Tesque, 21015-12) was drawn up from the storage of the syringe via the nozzle of the syringe. The plunger was then pulled up to the 100 $\mu$L mark without drawing up any solution, and normal laboratory air was filled. As a result, the ratio of the volume of air in the storage was set to 30 % to 80 %. After confirming that air was present on the plunger side and the D-PBS solution was present on the tip side of the nozzle, the air and solution were administered to the target.

(Observations after administration of liquid and gas)

The konjac jelly was sliced to a size of about 7 mm including the administration site and observed under a microscope. The agarose gel was prepared and administered in a 50 mL centrifuge tube and was visually observed from outside the centrifuge tube. The marshmallow was split lengthwise at the center of the administration site, and visual observation was performed.

Sections were prepared from the mice and pigs in the same manner as in Experiment 1 and observed under a microscope.

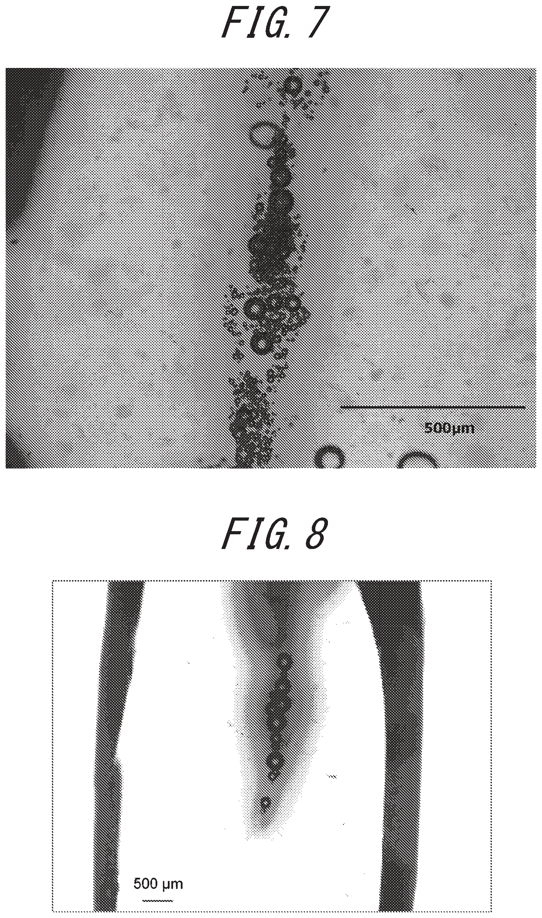

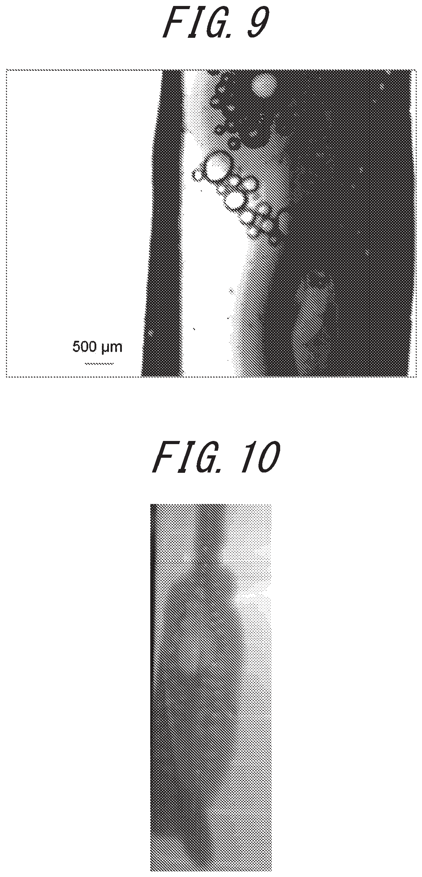

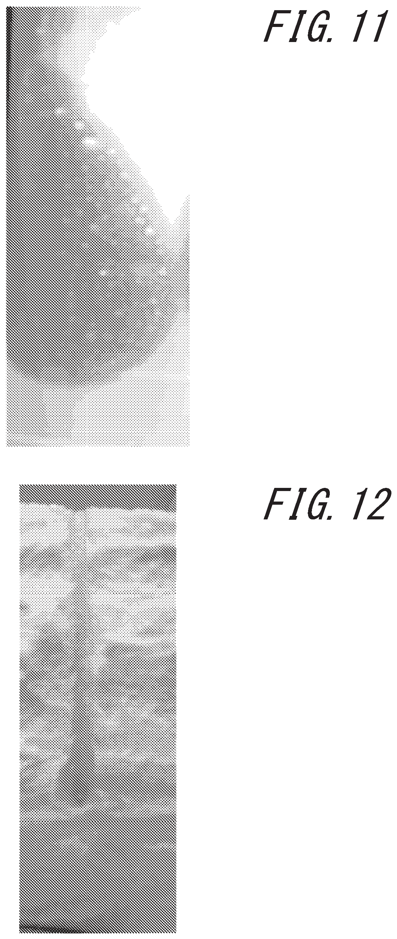

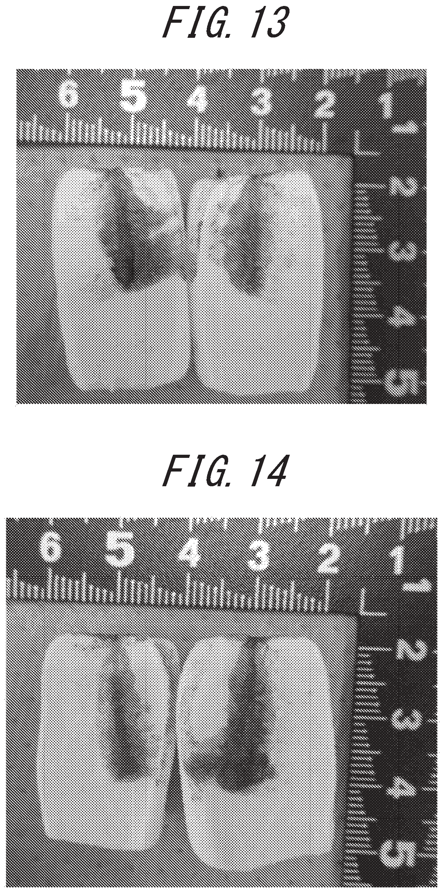

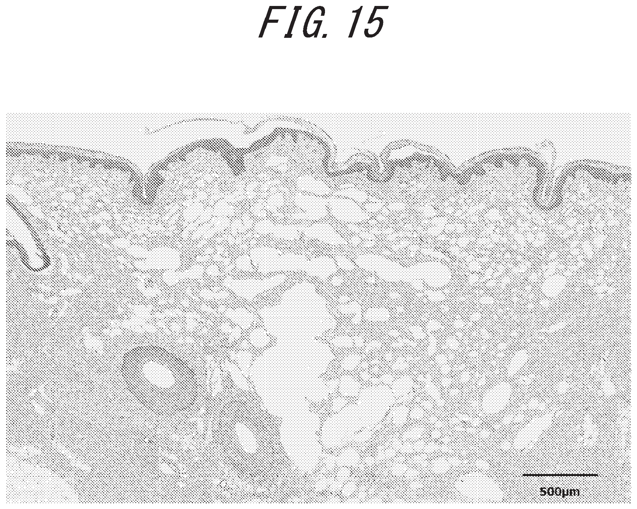

The observed images of each target are illustrated in FIGS. 7 to 15.

The formation of spaces was evaluated according to the following criteria.

A: A plurality of spaces was formed in the target. In the konjac jelly, agarose, and marshmallow, this refers to a state in which a plurality of bubble-like spaces are observed within the malachite green color.

B: One large space was formed in the target. In the konjac jelly, agarose, and marshmallows, this refers to a state in which one space is observed within the malachite green color.

C: No spaces were formed.

Table 2 illustrates the gas ratio, the results of measurements of hardness, elasticity, viscosity coefficient, viscoelastic modulus, and relaxation time using the Vesmeter, and the evaluation of space formation. The inguinal regions of a plurality of pigs were measured, and the results are labeled pig inguinal region 1 and pig inguinal region 2 for identification.

[Table 2]

**Table 2**

| Target | Gas ratio (%) | Hardness | Elasticity (kPa) | Viscosity coefficient (Pa·s) | Viscoelastic modulus (%) | Relaxation time (ms) | Space formation |
|---|---|---|---|---|---|---|---|
| 1 % Agarose gel | 50 | 27.7 | 245.2 | 218.6 | 47.8 | 0.89 | A |
| 1 % Agarose gel | 60 | 27.7 | 245.2 | 218.6 | 47.8 | 0.89 | A |
| 1 % Agarose gel | 80 | 27.7 | 245.2 | 218.6 | 47.8 | 0.89 | A |
| Pig inguinal region 1 | 50 | 4.4 | 109.6 | 245.6 | 58.6 | 2.27 | A |
| Pig inguinal region 1 | 30 | 4.4 | 109.6 | 245.6 | 58.6 | 2.27 | A |
| Konjac jelly | 50 | 5.0 | 76.0 | 235.4 | 60.9 | 3.11 | A |
| Konjac jelly | 60 | 5.0 | 76.0 | 235.4 | 60.9 | 3.11 | A |
| Konjac jelly | 80 | 5.0 | 76.0 | 235.4 | 60.9 | 3.11 | A |
| 0.7 % Agarose gel | 50 | 15.4 | 225.3 | 365.2 | 61.1 | 1.63 | A |
| 0.7 % Agarose gel | 60 | 15.4 | 225.3 | 365.2 | 61.1 | 1.63 | A |
| 0.7 % Agarose gel | 80 | 15.4 | 225.3 | 365.2 | 61.1 | 1.63 | A |

(continued)

| Target | Gas ratio (%) | Hardness | Elasticity (kPa) | Viscosity coefficient (Pa·s) | Viscoelastic modulus (%) | Relaxation time (ms) | Space formation |
|---|---|---|---|---|---|---|---|
| Pig inguinal region 2 | 50 | 3.2 | 102.7 | 276.3 | 62.6 | 2.69 | A |
| Pig inguinal region 2 | 30 | 32 | 102.7 | 276.3 | 62.6 | 2.69 | A |
| Pig abdomen (with air) | 50 | 2.6 | 123.8 | 276.9 | 63.0 | 2.25 | A |
| Pig back | 50 | 20.6 | 274.6 | 428.6 | 64.2 | 1.61 | A |
| Pig abdomen (no air) | 50 | 1.6 | 91.8 | 280.7 | 65.7 | 3.09 | A |
| 0.5 % Agarose gel | 50 | 12.1 | 204.9 | 409.1 | 66.0 | 2.00 | A |
| 0.5 % Agarose gel | 60 | 12.1 | 204.9 | 409.1 | 66.0 | 2.00 | A |
| 0.5 % Agarose gel | 80 | 12.1 | 204.9 | 409.1 | 66.0 | 2.00 | A |
| Mouse flank | 50 | 3.3 | 116.6 | 324.4 | 70.0 | 2.90 | A |
| Marshmallow | 50 | 1.3 | 103.4 | 453.1 | 81.9 | 4.39 | B |
| Marshmallow | 60 | 1.3 | 103.4 | 453.1 | 81.9 | 4.39 | B |
| Marshmallow | 80 | 1.3 | 103.4 | 453.1 | 81.9 | 4.39 | B |

As illustrated in Table 2, spaces were formed in the samples having a viscoelastic modulus at 25 °C of 47 % or more and 82 % or less and a relaxation time of 0.89 ms or more and 4.39 ms or less.

**Claims**

1. An injector for injecting a liquid and a gas into a target from an injector body without performing injection through a predetermined structure in a state in which the predetermined structure is inserted into the target, the injector comprising:

   a storage configured to store the liquid and the gas;
   a nozzle in communication with the storage and comprising an ejection port for ejecting the liquid and the gas toward the target; and
   a pressurizer configured to pressurize the liquid and the gas stored in the storage to eject the liquid and the gas from the ejection port toward the target when the pressurizer is activated,
   wherein a ratio of a volume of the gas to a total volume of the liquid and the gas is more than 60 % and 80 % or less.

2. The injector according to claim 1, wherein the gas is air.

3. The injector according to claim 1 or 2, wherein injection into the target is injection into the target by jet injection.

4. The injector according to claim 1 or 2, wherein an ejection speed of the liquid and the gas exceeds 83.3 μL/s.

5. The injector according to claim 1 or 2, wherein the liquid contains a biofunctional substance.

6. The injector according to claim 5, wherein the biofunctional substance is at least one selected from the group consisting of nucleic acids, peptides, proteins, and low molecular weight compounds.

7. An injector for reducing interstitial fluid accumulation in a target,
the injector injecting a liquid and a gas into a target from an injector body without performing injection through a predetermined structure in a state in which the predetermined structure is inserted into the target, the injector comprising:

   a storage configured to store the liquid and the gas;
   a nozzle in communication with the storage and comprising an ejection port for ejecting the liquid and the gas toward the target; and
   a pressurizer configured to pressurize the liquid and the gas stored in the storage to eject the liquid and the gas from the ejection port toward the target when the pressurizer is activated.

8. The injector according to claim 7, wherein a ratio of a volume of the gas to a total volume of the liquid and the gas is 30 % or more and 80 % or less.

9. The injector according to claim 7 or 8, wherein the gas is air.

10. The injector according to claim 7 or 8, wherein injection into the target is injection into the target by jet injection.

11. The injector according to claim 7 or 8, wherein an ejection speed of the liquid and the gas exceeds 83.3 $\mu$L/s.

12. The injector according to claim 7 or 8, wherein the liquid contains a biofunctional substance.

13. The injector according to claim 12, wherein the biofunctional substance is at least one selected from the group consisting of nucleic acids, peptides, proteins, and low molecular weight compounds.

14. A method for forming a space using an injector,
the injector injecting a liquid and a gas into a target from an injector body without performing injection through a predetermined structure in a state in which the predetermined structure is inserted into the target, the injector comprising:

   a storage configured to store the liquid and the gas;
   a nozzle in communication with the storage and comprising an ejection port for ejecting the liquid and the gas toward the target; and
   a pressurizer configured to pressurize the liquid and the gas stored in the storage to eject the liquid and the gas from the ejection port toward the target when the pressurizer is activated,
   the method comprising:

      storing the liquid and the gas in the storage;
      pressurizing the liquid and the gas stored in the storage; and
      injecting the liquid and the gas into the target,
      wherein an injection site of the target has a viscoelastic modulus of 47 % or more and 82 % or less at 25 °C.

15. The method for forming a space according to claim 14, wherein the injecting is injecting into the target by jet injection.

16. The method for forming a space according to claim 14 or 15, wherein a ratio of a volume of the gas to a total volume of the liquid and the gas is 30 % or more and 80 % or less.

17. The method for forming a space according to claim 14 or 15, wherein the gas is air.

18. The method for forming a space according to claim 14 or 15, wherein an ejection speed of the liquid and the gas exceeds 83.3 $\mu$L/s.

19. The method for forming a space according to claim 14 or 15, wherein a relaxation time when viscoelasticity is measured at 25 °C at an injection site of the target is 0.89 ms or more and 4.39 ms or less.

20. A method for reducing interstitial fluid accumulation in a target using an injector,
the injector injecting a liquid and a gas into a target from an injector body without performing injection through a predetermined structure in a state in which the predetermined structure is inserted into the target, the injector comprising:

a storage configured to store the liquid and the gas;
a nozzle in communication with the storage and comprising an ejection port for ejecting the liquid and the gas toward the target; and
a pressurizer configured to pressurize the liquid and the gas stored in the storage to eject the liquid and the gas from the ejection port toward the target when the pressurizer is activated,
the method comprising:

storing the liquid and the gas in the storage;
pressurizing the liquid and the gas stored in the storage; and
injecting the liquid and the gas into the target.

21. The method according to claim 20, wherein the target is a mammal other than a human.

22. The method according to claim 20 or 21, wherein the injecting is injecting into the target by jet injection.

23. The method according to claim 20 or 21, wherein a ratio of a volume of the gas to a total volume of the liquid and the gas is 30 % or more and 80 % or less.

24. The method according to claim 20 or 21, wherein the gas is air.

25. The method according to claim 20 or 21, wherein an ejection speed of the liquid and the gas exceeds 83.3 μL/s.

26. The method according to claim 20 or 21, wherein the liquid contains a biofunctional substance.

27. The method according to claim 26, wherein the biofunctional substance is at least one selected from the group consisting of nucleic acids, peptides, proteins, and low molecular weight compounds.

28. The method according to claim 20 or 21, further comprising injecting cells or a solution that contains a biofunctional substance into a space formed by the injecting of the liquid and the gas.

# FIG. 1

FIG. 2

FIG. 3

FIG. 4

## FIG. 5

500μm

## FIG. 6

## *FIG. 7*

500µm

## *FIG. 8*

500 µm

## FIG. 9

500 μm

## FIG. 10

FIG. 11

FIG. 12

## FIG. 13

## FIG. 14

# FIG. 15

500μm

## INTERNATIONAL SEARCH REPORT

| International application No. |
|---|
| **PCT/JP2024/024991** |

### A. CLASSIFICATION OF SUBJECT MATTER

*A61M 5/303*(2006.01)i; *A61K 35/12*(2015.01)i; *A61K 45/00*(2006.01)i; *A61P 43/00*(2006.01)i
FI:  A61M5/303; A61K45/00; A61P43/00 111; A61P43/00 171; A61P43/00 105; A61K35/12

According to International Patent Classification (IPC) or to both national classification and IPC

### B. FIELDS SEARCHED

Minimum documentation searched (classification system followed by classification symbols)

A61M5/303; A61K35/12; A61K45/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2020/116353 A1 (DAICEL CORP.) 11 June 2020 (2020-06-11)<br>paragraphs [0012], [0019], [0023]-[0025], [0051]-[0055], fig. 1 | 1-6 |

☐ Further documents are listed in the continuation of Box C.  ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **19 September 2024** | **01 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

# EP 4 751 750 A1

## INTERNATIONAL SEARCH REPORT

**Box No. III**    **Observations where unity of invention is lacking (Continuation of item 3 of first sheet)**

This International Searching Authority found multiple inventions in this international application, as follows:

The claims are classified into the following three inventions.
Invention 1: Claims 1-6
    Document 1 sets forth
    a syringe (1) for injecting liquid and gas into an object from a syringe body (6), when a prescribed structure has been inserted into the object, without performing the injection via the prescribed structure, the syringe being provided with
    an accommodating part (32) in which the liquid and gas are accommodated,
    a nozzle (31) that is in communication with the accommodating part (32), and has an emission port (31a) for emitting the liquid and gas toward the object, and
    pressurizing parts (4, 5, 7) for emitting the liquid and gas from the emission port (31a) toward the object during operation by pressurizing the liquid and gas accommodated in the accommodating part (32),
    wherein
    the proportion of the volume of gas with respect to the total volume of liquid and gas is 70% (see paragraph [0052])
(in particular, see paragraphs [0012], [0019], [0023]-[0025], [0051]-[0055], fig. 1). Since claims 1-2 are deprived of novelty in light of document 1, said claims do not have a special technical feature. Moreover, claim 3 is merely a simple design change of document 1, and thus does not have a special technical feature. As such, claims 1-3 are classified as invention 1.
    In addition, claims 4-6 depend from claim 1 and are inventively linked with claim 1; thus, said claims are classified as invention 1.

Invention 2: Claims 7-13 and 20-28
    Claims 7-13 and 20-28 share with claim 1, classified as invention 1, the common technical feature of
"a syringe for injecting liquid and gas into an object from a syringe body, when a prescribed structure has been inserted into the object, without performing the injection via the prescribed structure, the syringe being provided with
    an accommodating part in which the liquid and gas are accommodated,
    a nozzle that is in communication with the accommodating part, and has an emission port for emitting the liquid and gas toward the object, and
    pressurizing parts for emitting the liquid and gas from the emission port toward the object during operation by pressurizing the liquid and gas accommodated in the accommodating part."

However, on comparison with the content of the disclosure in document 1 (in particular, see paragraphs [0012], [0019], [0023]-[0025], [0051]-[0055], fig. 1), said technical feature does not make a contribution over the prior art, and thus cannot be said to be a special technical feature. Moreover, there are no other same or corresponding special technical features between claims 7-13 and 20-28 and claim 1.
    Furthermore, claims 7-13 and 20-28 do not depend from claim 1. In addition, claims 7-13 and 20-28 are not substantially identical to or similarly closely related to any of the claims classified as invention 1.
    As such, claims 7-13 and 20-28 cannot be classified as invention 1.
    Since claims 7-13 and 20-28 have the special technical feature of "reducing interstitial fluid accumulated in the object," said claims are classified as invention 2.

Invention 3: Claims 14-19
    Claims 14-19 share with claim 1, classified as invention 1, and claims 7 and 20, classified as invention 2, a common technical feature of
"a syringe for injecting liquid and gas into an object from a syringe body, when a prescribed structure has been inserted into the object, without performing the injection via the prescribed structure, the syringe being provided with
    an accommodating part in which the liquid and gas are accommodated,
    a nozzle that is in communication with the accommodating part, and has an emission port for emitting the liquid and gas toward the object, and
    pressurizing parts for emitting the liquid and gas from the emission port toward the object during operation by pressurizing the liquid and gas accommodated in the accommodating part."

Form PCT/ISA/210 (continuation of first sheet) (July 2022)

## INTERNATIONAL SEARCH REPORT

| International application No. |
| --- |
| **PCT/JP2024/024991** |

**Box No. III      Observations where unity of invention is lacking (Continuation of item 3 of first sheet)**

However, on comparison with the content of the disclosure in document 1 (in particular, see paragraphs [0012], [0019], [0023]-[0025], [0051]-[0055], fig. 1), said technical feature does not make a contribution over the prior art, and thus cannot be said to be a special technical feature. Moreover, there are no other same or corresponding special technical features between claims 14-19 and claim 1 or claims 7 and 20.

Furthermore, claims 14-19 do not depend from any of claims 1, 7, or20. In addition, claims 14-19 are not substantially identical to or similarly closely related to any of the claims classified as invention 1 or 2.

As such, claims 14-19 cannot be classified as either invention 1 or 2.

Since claims 14-19 have the special technical feature of "a cavity-forming method that uses a syringe, the forming method comprising a step for accommodating the liquid and gas in the accommodating part, a step for pressurizing the liquid and gas accommodated in the accommodating part, and a step for injecting the liquid and gas into the object, wherein the viscoelastic modulus of the injection site in the object at 25°C is 47% to 82%," said claims are classified as invention 3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-6**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2024/024991**

| Patent document cited in search report | Publication date (day/month/year) | Patent family member(s) | Publication date (day/month/year) |
|---|---|---|---|
| WO 2020/116353 A1 | 11 June 2020 | US 2022/0017847 A1 paragraphs [0036], [0046]-[0049], [0055]-[0058], [0102]-[0107], fig. 1 EP 3892711 A1 CN 113166696 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004358234 A
- US 20050010168 A1
- WO 2022149550 A1
- WO 2001031282 A1
- JP 2003025950 A

**Non-patent literature cited in the description**

- *Can. Oncol. Nurs. J.*, 2012, vol. 22 (4), 222-34